# EUROPEAN PATENT SPECIFICATION

(11) **EP 2 928 405 B1**
(45) Date of publication and mention of the grant of the patent: **10.10.2018**
(21) Application number: 13824179.9
(22) Date of filing: 27.11.2013
(51) Int. Cl.: A61B 34/10, A61B 34/20, A61B 34/00, A61B 90/00, A61B 18/00, A61B 18/14, A61B 5/06, A61B 6/00, A61B 6/12, A61B 90/10

(54) **POSITIONING TOOL**
POSITIONIERUNGSINSTRUMENT
OUTIL DE POSITIONNEMENT

(30) Priority: 10.12.2012 EP 12306553
(43) Date of publication of application: 14.10.2015
(73) Proprietor: Koninklijke Philips N.V., 5656 AE Eindhoven (NL)
(72) Inventor: FLORENT, Raoul, 5656 AE Eindhoven (NL)
(74) Representative: Damen, Daniel Martijn
(86) International application number: PCT/IB2013/060434
(87) International publication number: WO 2014/091347

(56) References cited:
- JP-A- 2008 167 793

## Description

### FIELD OF THE INVENTION

The present invention relates to an apparatus for supporting medical device positioning, a method for medical device positioning, to an imaging system, to a computer program element and to a computer readable medium.

### BACKGROUND OF THE INVENTION

One of the most prevalent medical conditions across the world is hypertension. Drug makers have responded to this and developed a large range of anti-hypertensive drugs for treatment.

Unfortunately, certain patients do not respond to such medication. It has been found that in some of those non-responsive patients their sympathetic nervous system operates to maintain the body in a constant state of "fight-or-flight", i.e., in a state of stress. Maintaining such a state includes precisely keeping blood pressure at a relatively high level. To this end, the sympathetic nervous system transmits signals via nervous tissue to the body's kidneys to instruct same to produce high quantities of renin. This enzyme is used in human metabolism to regulate, for example, arterial constriction across the body to so cause and maintain high blood pressure.

To address this situation an interventional procedure called renal denervation has been developed. In renal denervation, a specially adapted energizable catheter is introduced into a renal artery. The catheter is used to at least partially de-activate nervous tissue that lies in the artery's wall and leads in and out of the respective kidney. In other words the communication between the sympathetic nervous system and the kidney is thereby curtailed to drive down renin production and, ultimately, blood pressure.

It has been found, however, that operating those denervation systems or catheters or similar interventional equipment is at times difficult. This may also lead to treatment results that fall short of expectations.

JP 2008-167793 describes an apparatus for supporting surgery through realistic representation in a displayed image of a simulation of a surgical cut. The simulation is based on three-dimensional image data of a subject.

### SUMMARY OF THE INVENTION

There may therefore be a need for an apparatus to support medical personnel during denervation or similar interventional procedures.

The object of the present invention is solved by the subject matter of the independent claims where further embodiments are incorporated in the dependent claims. It should be noted that the following described aspect of the invention equally applies to the method for medical device positioning, to the imager system, to the computer program element and to the computer readable medium.

According to a first aspect of the invention there is provided an apparatus for positioning a medical device (such as a denervation catheter or others). The apparatus comprises:
an input port for receiving i) an X-ray projection image of an object and ii) a signal indicative of a current application position for or of a medical device in or around said object;
a user interface generator configured to generate for display on a screen a marker overlaid on said image, the marker indicating a next application position for the device at a first pre-determined distance from said current or previous application position, the generator responsive to overlay a second marker for a second position at a second pre-determined distance from the next position upon receipt, at the input port, of a signal indicative of said second position.

According to one embodiment, the application positions can be thought of as points of action, that is, a position where the medical device is used to deliver a certain task. The apparatus may be used during a denervation intervention, where a tip of a denervation catheter needs to be precisely positioned in a specific pattern to repeatedly and point-wisely char or burn (thereby ablating) nervous tissue in a renal artery for example. The respective makers then mark-up the positions of the ablation sites, that is, where the denervation catheter has been applied ("current" or "previous") or where the catheter is intended to be applied next. The one or more markers, which are generated by the user-interface generator, are virtual markers that are superimposed on a current X-ray projection image. According to one embodiment, it is a respective single maker for any one of the consecutive next positions that is displayed as the medical device moves from one position to the next: In other words, the first marker is erased once the second marker is displayed. However in other embodiments, the two markers are displayed together so that at any time there are always exactly two markers displayed and this embodiment can be extended to display all or the latest *n* (*n≥*2) previous points of action positions.

In one embodiment, there is also a tracker marker that tracks across a sequence of images the actual current position of the device, taken for example, relative to the catheter's tip. This marker for current tip position is in general different from the markers for the device application positions, for example, when the device travels between the previous ablation site towards the next. This tracker marker is displayed alongside the one or more action point position markers. In other words, the apparatus affords a dynamic target indication for the operation of the medical device relative to current point of action/position.

The image shows, when displayed, at least a projection view or projection view profile ("footprint") of the catheter and guides the operator to the next ablation point given a current ablation point. This obviates the need to guess the next position for the catheter tip by eye and it also obviates the error-prone and cumbersome use of physical rulers or gauges that are placed with the patient during the X-ray acquisition. The markers can be overlaid in a great many shapes such as crosshair reticules or simple line segments. As the operator progresses the denervation catheter through the renal artery, a sequence of markers is produced.

The apparatus as proposed herein helps operator "zero-in", i.e., visually focus, without memorizing effort, on the next target position where the next ablation is to be applied. Operator can forget about the previous ablation point and can fully focus attention on the next position. The apparatus helps the operator to quickly and conveniently achieve a uniform ablation point pattern.

According to one embodiment the marker sequence progresses along a direction that is aligned with the position of the relevant organ (for example the renal artery) and/or the medical device. Knowledge of the anatomy of interest and the imager's geometry that is used to acquire fluoroscopy or angiographic images during the intervention can be used. For example, in the setting of a renal denervation procedure, the relevant renal artery is normally shown across the image horizontally. According to this embodiment the sequence of markers progresses likewise horizontally thereby following the dimension of the relevant anatomic structure. However, in other embodiments where for example the medical device extends along a curved path, the user interface generator automatically adapts to this curved situation and therefore the sequence of markers progresses along a curved extremity of the relevant organ footprint and/or curved footprint of the medical device.

According to one embodiment there is also a virtual gauge or ruler generated and displayed along the one or plurality of markers. "Gauge" as used herein includes but is not limited to graphical representations of a ruler, straightedge or, in particular for curvilinear applications, of a measuring tape, ribbon or cord. This allows the operator to read off in real terms the distances or assists him or her in positioning an interventional tool such as a denervation catheter.

According to one embodiment the user interface generator is configured to automatically align the virtual gauge overlaid with the device or object orientation and or to automatically scale the length and a graduation on the gauge to a length of the object or device. The in-image virtual gauge is a device and/or ROI adapted and digitally represented as on-screen measurement and positioning aid. In one embodiment the gauge is automatically or at least-semi-automatically scaled, oriented and positioned in respect of the footprint of the device and/or organ of interest. Alignment is automatic in that alignment is effected upon receipt of the image. In some embodiments the alignment operation (or all or certain of the user interface generator operations described herein) is also (or instead) semi-automatic in that the user interface generator also accepts input after the image with the markers/gauge is displayed and operates to modify markers/gauge based on said newly or later provided user input or is based on input queried from the imager, such as changes in the imaging geometry, e.g., table movements.

The ruler or gauge is virtual in the sense that it is a GUI widget or similar pixel information that is used to represent said ruler. Said representative pixel information is not related to any attenuation as would have been the case had, following current practice, a "real", physical ruler been placed with the patient during the X-ray image acquisition. In other words, such physical rulers or similar measuring devices are no longer required. The virtual gauge as proposed herein increases the comfort for the patient and allows for a more accurate operation of the denervation catheter. The virtual gauge may also be displayed as a virtual protractor in curvilinear applications. In this case a segmenter operates to extract from the image (for example a fluoroscopic X-ray image, also referred hereinafter as a "fluoro" or an angiogram also referred hereinafter as "angio) the relevant structure to compute an arc length along said structure in order to place the marker at the right "geodesic" distance. Precise positioning of the denervation catheter or similar interventional tools can be so achieved even in curvilinear environments.

In one embodiment, the overall on-screen length of the virtual gauge (or, for that matter, the overall length along which the markers are to run) is according to a pre-set value that relates to the examination type at hand. For instance, in renal denervations the average renal artery length is known and can be user supplied via suitable user input means or can be retrieved from a medical database upon the user specifying the exam and/or organ type.

According to one embodiment the markers and/or the ruler or gauge is interactive in that the operator can use a pointer tools such as a mouse to evoke additional clinically relevant information, for example clicking on the digital gauge and/or the marker so as to effect a pop-up of a GUI window to represent contextual information or offer contextual user interaction. In one embodiment the (by the apparatus) proposed next marker position can be user "edited", that is, changed by the user in order to take into account for instance the presence of calcifications in the vessel where an ablation is better avoided. There is a safety margin around the required ablation point clearance so the user has some leeway to make minor adjustments to the apparatus-indicated next ablation point position. If however the user attempts to adjust so as to violate said safety margin (if for example he or she attempts to set the ablation point too close to the previous one) an error message to this effect is issued. In one embodiment, the apparatus may issue even a "disable" signal to the denervation generator to prevent the user to carry out the denervation at that point.

According to one embodiment the graduations correspond to the required ablation point clearance, that is, the distance between any two immediately consecutive graduations on the ruler DR equals the required ablation point clearance in pixel terms.

According to one embodiment the distance between any two immediate graduations distance correspond to a user selectable physical scale such as mm, cm, and inches etc., again expressed on-screen on the virtual ruler in pixel terms.

According to one embodiment the signal that is indicative of the respective current catheter position is obtained by interfacing with the particular denervation tool used for the intervention. According to one embodiment however, no such interfacing is required but the system is solely based on the sequence of images to determine the current denervation point. The scaling of the marker distance and the graduations on the digital ruler so adjusted that moving the catheter tip position according to those markers will affect the real positioning at the desired distances. The on-screen distances between the markers or between the graduations are to be expressed in or related to a natural distance unit (for example millimeters), and not in pixels since it is the actual physical distances between the ablation points in-situ that are of interest. In order to translate from physical distance to pixels distance (markers and gauge graduations are viewed in the pixel domain) a unit transformation can be defined. According to one embodiment, this mm/pixel relationship can be derived by considering the footprint (projection view) in one of the images of a characteristic portion of the device such as the catheter tip or guidewire etc. which can be assumed to be known. The device's tip portion footprint is segmented ("extracted"), that is, identified, in the image based on knowledge of the tip's physical dimensions and shape which can be obtained from manufacturer's product specification. This knowledge then provides a natural reference to translate how many pixels there are to the length unit (for example millimeter) chosen. Another embodiment harnesses the X-ray imager's imaging geometry used when acquiring the projection image to approximate the mm/pixel relationship. If it is assumed that the region of interest, for example a relevant renal artery, is at the iso-center of the imager, the mm-pixel correspondence can be deduced from the X-ray beam divergence caused by the selected SID (X-ray-source-detector-distance). This relationship may then be used as an approximation for the mm/pixel relationship throughout the whole vessel under consideration.

Referring back to the renal denervation context, the user controlled progression of catheter during the denervation is monitored by a sequence of live fluoros IM that are displayed in said sequence on screen M. Controller operates so that the one or more marker is overlaid on each of said life fluoros as they are displayed. Said operation of controller thereby ensures user can observe the marker throughout the live fluoro sequence and better control position of the catheter tip as it is made to progress in the pull-back phase through the renal artery from a previous ablation point to the next ablation point positions, one by one, where the next ablations are to be applied at the respective positions on the artery wall. A typical denervation intervention includes applying about 4-6 point-wise ablations and a respective marker for each of the ablation point positions is sequentially displayed on the respective fluoros when the catheter tip T is approaching the respective positions. Consecutive ablation position markers are displayed at the required clearance distance which is user configurable is usually in the order of 5mm for renal a denervation as observed earlier. In other words, during the course of the intervention controller operates so that a sequence of ablation position markers is displayed as "propagating" across the image plane in a configurable direction d. The propagation direction of the ablation markers is automatically aligned with the footprint of the catheter or the footprint of the organ of interest.

Optionally or automatically at least one of the markers is a bar or line element so displayed so as to extend across said lead line. The lead line may be a bar element or may extend as a ribbon or band across the image or may indeed be a curvilinear band or curve for curvilinear objects and or devices.

Although herein the use of apparatus has been explained with reference to renal denervation and a respective catheter it is understood that the proposed apparatus may be put to good use in other contexts as well where precise positioning is required. For example application of the proposed apparatus is also envisaged in tumor ablation interventions using with RF (radio frequency) needles.

According to one embodiment the X-ray projection image is acquired by an X-ray imager. The alignment and or maker positioning operation of interface generator is based on a segmentation operation of an X-ray footprint of medical device in said image or of an X-ray footprint of the object in said image or in an object X-ray image (angio).

According to one embodiment the device issues said second signal whilst the device is or operates at the respective position or wherein said second signal is issued upon the device reaching said next position.

According to one embodiment the at least one of the markers is a solid, dashed or dotted line or bar segment displayed so as to extend or run across said direction or wherein at least one of the two markers is displayed as any one or a combination of a cross-hair symbol, a chevron symbol, a circle, a dot. Of course other graphical symbols that help a human user to easily discern positions on screen may also be used.

### BRIEF DESCRIPTION OF THE DRAWINGS

Exemplary embodiments of the invention will now be described with reference to the following drawings wherein:
Fig 1 shows a view of a renal artery during a denervation intervention;
Fig 2 shows an arrangement for supporting a denervation intervention;
Fig 3 is a close-up view of Fig 1;
Fig 4 is a detailed view of Fig 2 showing a graphical user interface;
Fig 5 is a flow chart for a method of supporting positioning of a medical device.

### DETAILED DESCRIPTION OF EMBODIMENTS

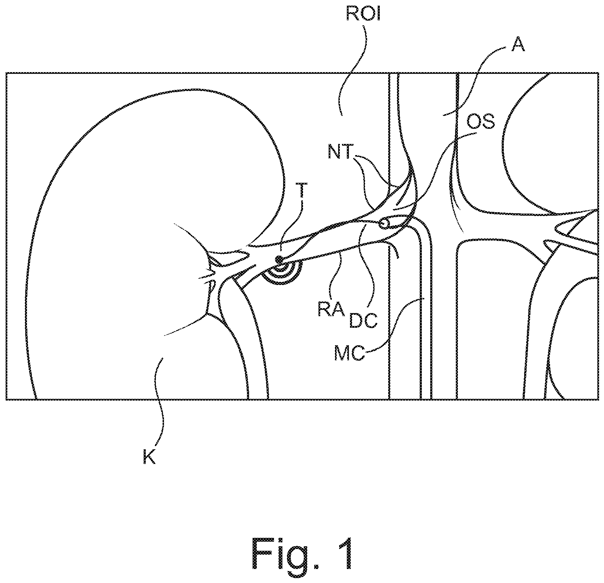

With reference to Figure 1 there is shown a schematic view on the relevant anatomic situation ROI in a renal denervation in respect of a human or animal body. A renal feeder artery RA branches off from aorta A at an ostium OS. Renal artery RA forms the conduit via which kidney K is supplied with blood. Vessel walls or renal artery RA are enmeshed with nervous tissue NT via which the central nervous system communicates information to kidney K to control inter alia kidney K's renin production. The view also shows an in-situ position of a denervation catheter DC having an energizable tip T schematically indicted in Fig 1 by a sequence of emanating circular arcs. The operation of said catheter denervation DC will be explained in more detail below.

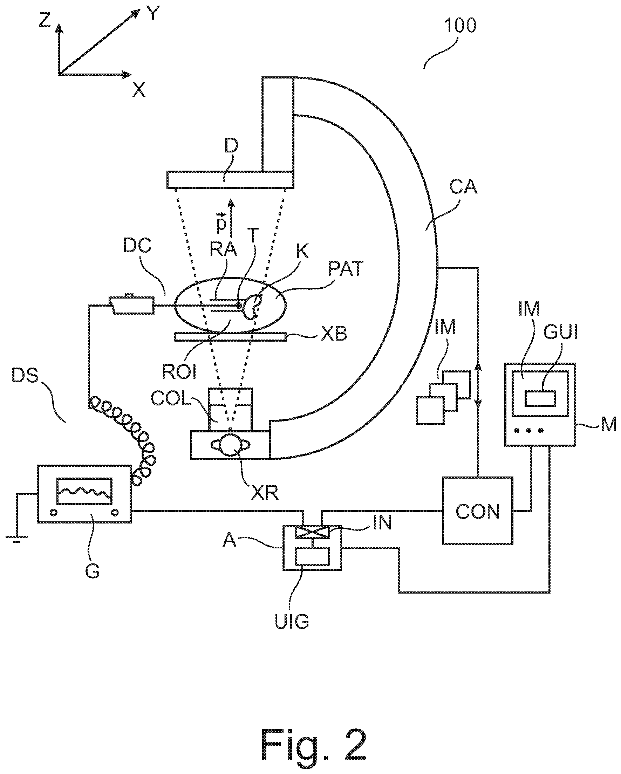

Figure 2 shows an arrangement to support a renal denervation procedure on the human or animal body. The arrangement includes an X-ray imager 100 and a denervation system DS. Figure 2 shows imager 100 of the C-arm type however it is understood that other imager constructions may also be put to use.

Imager 100 includes a rigid C-arm CA having affixed thereto at one of its ends a detector D and to the other an X-ray tube XR and a collimator COL (hereinafter together referred to as the C-X-assembly). X-ray tube XR operates to generate and emit a primary radiation X-ray beam *p* whose main direction is schematically indicted by vector *p.* Collimator COL operates to collimate said X-ray beam in respect of a ROI.

The position of the arm CA is adjustable so that the projection images can be acquired along different projection directions p. The arm CA is rotatably mounted around the examination table XB. The arm CA and with it the CX assembly is driven by a stepper motor or other suitable actuator.

Overall operation of imager 100 is controlled by operator from a computer console CON. Console CON is coupled to screen M. Operator can control via said console CON any one image acquisition by releasing individual X-ray exposures for example by actuating a joy stick or pedal or other suitable input means coupled to said console CON.

During the intervention and imaging an examination table XB (and with it patient PAT) is positioned between detector D and X-ray tube XR such that the lesioned site or any other related region of interest ROI is irradiated by primary radiation beam PR.

Broadly, during an image acquisition the collimated X-ray beam PR emanates from X-ray tube XR, passes through patient PAT at said region ROI, experiences attenuation by interaction with matter therein, and the so attenuated beam PR then strikes the detector D's surface at a plurality of the detector cells. Each cell that is struck by an individual ray (of said primary beam PR) responds by issuing a corresponding electric signal. The collection of said signals is then translated by a data acquisition system ("DAS" - not shown) into a respective digital value representative of said attenuation. The density of the organic material making up the ROI determines the level of attenuation. High density material (such as bone) causes higher attenuation than less dense materials (such as the vessel tissue). The collection of the so registered digital values for each (X-)ray are then consolidated into an array of digital values forming an X-ray projection image for a given acquisition time and projection direction.

The denervation system DS includes a generator G (for generating radio frequency (RF) energy) in communication with the denervation catheter DC. The denervation procedure is an endovascular procedure similar to angioplasty. A user (also referred to herein as an operator, for example, an interventional radiologist) inserts denervation catheter DC for example through a femur artery in the upper thigh of a patient PAT and threads it into the renal artery RA. The catheter DC includes a flexible guidewire. The catheter is supported by a micro-catheter MC that is placed at ostium OS. Once the catheter DC's tip T is at the desired position inside the renal artery RA, the tip T is made to contact the inner arterial wall and denervation catheter DC is energized by activation of a generator G to deliver a controlled amount of radio frequency energy to effect a point-wise charring or ablation of nervous tissue at the point where catheter tip T currently resides and contacts the vessel' wall. The renal denervation procedure is image controlled in that imager 100 is operated to acquire as sequence of "live" fluoroscopic X-ray projection images IM ("fluoros") or angiograms ("angios") during the denervation procedure.

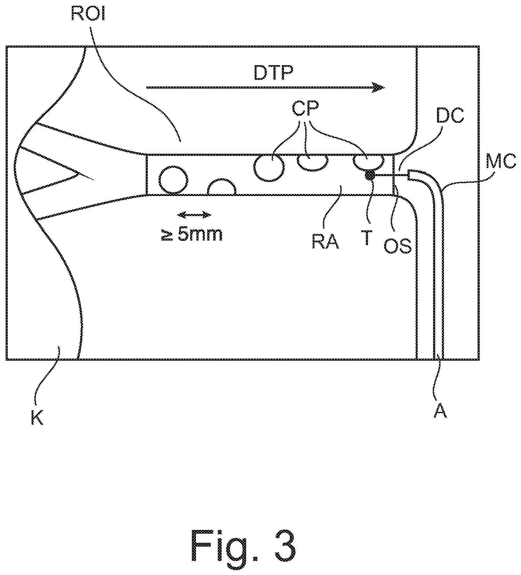

Figure 3 shows a close-up of the situation of Figure 1 to more clearly explain the denervation procedure. Catheter DC's tip T is firstly threaded through aorta A into renal artery RA and is then positioned at kidney K, that is, distal from said aorta A. In an image controlled distal-to-proximal pull back sequence (shown as arrow DTP), catheter DC is then pulled by the operator away from kidney K and towards the aorta A whilst tip T maintains contact with the renal artery's RA wall and whilst tip T is made to sweep out circles on the inside renal artery RA wall. Tip T therefore traces out a helix on the inner artery wall and catheter tip T is made to reside for a while at certain ablation or char positions CP to point-wisely deliver the RF energy to effect the ablation of nervous tissue NT at respective ones of those points, one point at a time. In this manner whilst catheter DC is withdrawn in a "stop- and-go" fashion in the pull-back, an ideally uniform pattern of ablation or char points CP is applied to the inner wall of the renal artery RA to effect treatment. It has been found that the effectiveness of the denervation procedure rests largely on the uniformity of the char/ablation point pattern that was applied to the renal artery wall RA. The more uniform the spacing between each neighboring char point CP the more effective the drop in blood pressure. The discrete, individual, point-wise RF ablation operations last for about 2 minutes at each ablation point and 4 to 6 ablation points separated longitudinally and circumferentially are achieved for each renal artery. The ablation points CP are spaced apart a minimum of 5 mm (referred hereinafter as the "required ablation point clearance") as measured longitudinally along the renal artery RA axis and are applied during the pullback from distal (kidney K) to proximal (aorta A) in a circumferential manner. A control angiogram is performed after the procedure. In one embodiment it is envisaged that the operator supplies the required (minimum) distance or clearance between two consecutive or neighboring ablation points CP that is to be respected and used for the denervation procedure. The user can specify the required ablation point clearance via keystroke input or via a GIU widget such as a drop-down menu or other graphical input arrangement or otherwise.

Because of the opacity of the catheter DC, its footprint DCFP is clearly visible in a fluoro whereas the outline of vessel RA is not. If the outline of the vessel is desired or needs to be segmented, a volume of contrast agent is administered to so at least temporarily confer opacity to the renal artery RA and imager 100 is operated to acquire one or more angios. An angio is a projection image whose pixel information is capable of encoding (that is, representing) the footprint or projection view of the artery To display on screen M the current position of the catheter DC (measured by the position of tip T) in relation to the surrounding renal artery suitable road mapping techniques can be used. A fluoro IM and a roadmap graphics element can then be displayed together on screen M or the angio is displayed instead of the current fluoro for as long as the operator wishes to actually see the vessel's footprint. For the purpose of a denervation, the artery RA determines the path that the catheter DC is to travel. However in other contexts than the exemplary denervation described herein, other organ footprints or landmarks may be used to define the medical device DC's operation path.

In order to help the operator to correctly position the catheter tip T in the desired uniform pattern where the char points are to be applied one at a time, the arrangement in Fig 2 also includes an apparatus A the operation of which will be explained in more detail below. Broadly speaking, apparatus A generates, based on current projection image IM, and a signal representative of the current ablation position of catheter tip T or a previous ablation position, a graphical user interface GUI which is then shown (on monitor M) overlaid on said current fluoro IM or angio or on any one of a sequence of live fluoros.

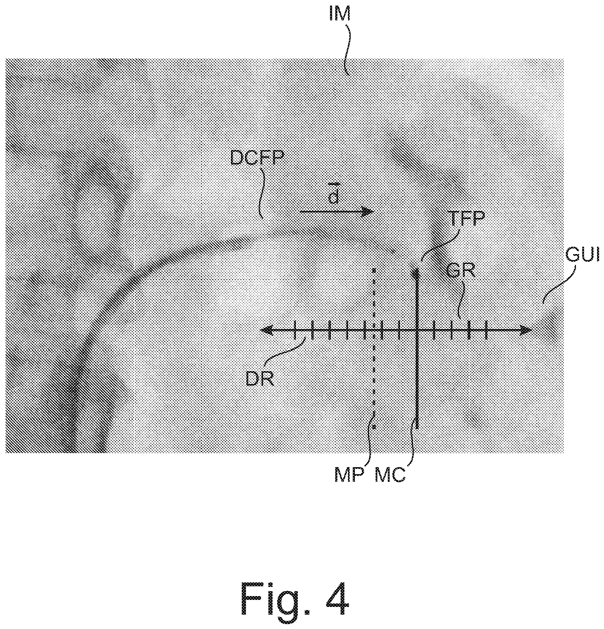

Figure 4 shows a more detailed view of said graphical user interface GUI overlaid on a current projection image IM. Projection image IM shows footprints DCFP of the denervation catheter DC and the footprint TFP of the catheter's energizable tip T. As mentioned earlier, the renal artery footprint is not shown for lack of radio opacity but at least the artery's orientation and extension is indirectly derivable from the device DC's footprint DCFP or of its guidewire.

According to one embodiment the graphical user interface GUI includes, overlaid on the said image IM, a marker MC for the next target position for the ablation where the catheter tip position T is to be positioned for the next point-wise ablation operation.

According to one embodiment, there is also a marker MP for the position of the immediate previous ablation position, that is, where the ablation has just been applied.

According to one embodiment it is only the marker position MC of the next catheter tip position that is displayed so as to guide the operator where to position the catheter tip position next.

The situation in the "dual marker" embodiment as shown in Figure 4 is that where the previous ablation position is indicted and displayed by marker MP (shown in dashed lines) alongside with the next (intended) catheter tip target position for the next ablation indicated by MC (shown in solid lines). Fig 4 shows the situation where tip T just reached marker position M. In this dual marker embodiment, as soon as the catheter tip reaches the next target point, the graphical user interface GUI is updated and a switchover of markers occurs, that is, previous marker MP is erased, marker MC now becomes the new previous ablation position, and a new marker (not shown) pops up and is displayed to the right of marker MC at the required intra-ablation clearance and the cycle then repeats as the tip T (after the ablation at point MC is accomplished) continues its journey through the renal artery RA towards the new next ablation position. In this manner, only two markers MC, MP are shown at any given time, namely the previous one MP and at the next position MC, where the operator needs to position the catheter tip T position next.

However other embodiments are also envisaged with a longer "trailing end", that is, where along with the next catheter tip ablation position it is also previous ablation point positions (the previous n ≥2 one) that are displayed. This is in contrast to the single marker embodiment mentioned earlier, where at any time only a single marker MC is displayed, namely the one for the next ablation point that lies ahead. Said maker is observed by the user to hop across the screen M as the tip closed in on the respective position one-by-one.

The signal that triggers the marker change over, is, as described, the intersection of the tip T's footprint TFP with the position demarked by next ablation point marker MC. However in other embodiments said signal is furnished by operation of the denervation catheter DC as described in more detail below. In yet another embodiment, the signal is time based, that is, it issues once the current position of the tip T footprint TFP (which is tracked across a sequence of fluoros) resides at a given point for longer than a predefined time limit which is interpreted by the apparatus that the operator is carrying out an ablation at that point. More details in relation to the signals are described in more detail further below.

As shown in Figure 4 the markers MP, MC can be represented as simple horizontal line segments displayed in a color that is automatically adapted to stand out better to the eye given the current background of image IM's image plane where the marker is to be displayed. In other words, the coloring of the digital ruler and/or markers change in relation to the background color of the relevant image on which marker or digital ruler is overlaid. For instance, if the image plain background is dark the relevant portion digital ruler is shown in a lighter color and vice versa. In yet other words the visual appearance of the marker and/or digital ruler changes as the device progresses through the patient.

Markers may be displayed in other embodiments as circles, cross hair, reticules, etc. The markers are virtual, that is, they are defined by an artificial pixel pattern or symbology. In one embodiment the user interface generator UIG retrieves from a library of "pre-fabricated" widgets suitable primitives of which the markers are then composed. User interface generator UIG then effects display of the markers at the determined positions after a suitable scaling. In one embodiment, the markers MP, MC are placed in the image IM on a vessel RA footprint (e.g. as circle, or more generally a point-wise representation). In this case, a segmentation of the artery footprint is carried on a corresponding angio. However in situations where the device DC footprint itself is a good indicator for the relevant organ RA in questions (as is the case for the renal denervation), the markers are placed on/along the device DC footprint. Since as in renal denervation the path from distal to proximal, the catheter guidewire defines the path of the predicted marker locations. In this case it is then the wire DC footprint that is segmented which can be done on the fluoro itself, so no angio is required, that is, no segmentation of the vessel RA itself is required.

According to yet another embodiment the user interface GUI generated by apparatus A also includes overlaid alongside with the one or more position markers MP, MC a digital ruler or gauge DR so as to enable the operator to read off distances in real terms.

Operation of apparatus A will now be explained in more detail.

### Operation

Denervation catheter positioning tool A comprises an input port IN and as mentioned earlier a user interface generator UIG.

Apparatus A receives via input port IN a current projection image IM and a signal indicative of the current catheter tip position and/or current ablation position. There may also be embodiments with separate input ports for each.

Two basic embodiments are envisaged: In one embodiment the current ablation position is obtained by interfacing with the denervation system DS. In this embodiment, apparatus A includes a suitably arranged interface and a suitable signal within the denervation system is intercepted and is deemed indicative for the energizing of the catheter's tip T. In other words when the energizing signal is intercepted this is interpreted that the catheter tip has arrived at a desired ablation point. The segmenter can then use the current image to segment for the catheter's tip for example by pixel-grey-value thresholding. Of course evaluating pixel values in a color image is also envisaged to be included herein. Because the shape of the catheter's tip is known the segmentation of the catheter tip in the image plain X, Y can be obtained quickly, In the dual marker mode, said position is then forwarded to user interface generator UIG which responds by rendering and arranging for display of a widget for marker MP so as to intersect that current ablation position. The next ablation position MC is then displayed at the specified ablation point clearance along a direction d. The determination of said marker propagation direction (which is also used to orient the digital ruler DR in the image plane) is explained in more detail below.

In another embodiment there is also a security mechanism since an ablation might be aborted or interrupted (invalidated) if, for example, the user finds apposition of the tip against the vessel wall is not satisfactory. One should in practice wait for an "ablation-completed" OK signal. But the generator provides this information (meaning certain DS parameters such as impedance, energy, and temperature) remaining within certain limits for a given time (around 2 minutes). Those DS parameters can be retrieved by interrogation of the DS generator where those parameters are accurately monitored.

In the above embodiments, segmenter can be used if device DC footprint DCFP itself does not give sufficient clues on the path the device is to take or if device is to be used in a forward-tracking procedure (as opposed the backtracking procedure for the pull-back explained above in relation to the denervation procedure as shown in Fig. 3) where the path for the markers needs to be established first. In one embodiment a corresponding angio is used and vessel RA of interest is segmented and has, for example, one of its longitudinal boundaries or its center axis approximated by a spline curve. That curve then defines the propagation direction along which the ablation point markers MC, MP will be placed and "pop up" as the device progresses along that path. If the curve is not linear the required ablation point clearance is defined in terms of arc length.

In the other basic embodiment determination of the current ablation point position is achieved without interfacing with the denervation system DS. This embodiment operates solely on image processing techniques and image metadata. This image processing based embodiment involves a decision making step on whether the denervation catheter tip remained still for a pre-defined period of time, that is, the period of time it takes to conclude an ablation operation at a given point. The point-wise ablation time period is known and is usually in the order of about 2 minutes and is provided to the system as a setting parameter. However, the actual ablation at a point is not carried out fully under fluoroscopy monitoring but is interrupted once or more to keep patient dosage at bay so it is only the ablation start that is "on beam". To still make the determination of the ablation time with image processing technique "robust" enough, one must establish with sufficient confidence that the catheter tip DC has indeed remained still for at least the required ablation application time. To account for the interruptions of the fluoroscopy monitoring, the time stamps of the individual fluoro frames are evaluated. If the catheter tip DC is found to have remained at the same position during two consecutive image runs, one corresponding to the ablation operation start and one corresponding to the ablation operation end (or shortly after ablation operation termination, but still before the tip was actually moved), then the apparatus's decision logic concludes (by comparing tip position and time stamps) that a valid ablation has indeed occurred at the current position and the marker MC for the next ablation position point is then displayed as previously described herein.

During the denervation procedure and in one embodiment, the current position of the catheter tip T is tracked automatically across the sequence of fluoros using segmentation by pixel grey value thresholding. A marker is then overlaid on the respective fluoro at the respective image plane position.

As can be seen in Figure 4, as the catheter tip traces out its path through the renal artery, the markers MP, MC are likewise tracing out a path in said direction, referred herein as the propagation direction d. As indicated in Fig 4 by arrow d it is envisaged by the apparatus A as proposed herein that said direction is determined automatically from image features of the current image IM. The direction may be obtained by the footprint of the considered organ in question and or a footprint of the current medical device such as in this case the catheter DC. To effect this automatic determination of marker MP propagation, in one embodiment, the organ or device footprint is segmented in an initial image and a spline curve is fitted to the boundary of the segmentation. The tangential directions along said curve are then used to define the propagation direction of the ablation point markers for example by averaging said tangential direction. The directions may change for curved boundaries as mentioned earlier but may also be constant as is the case for renal artery RA that extends essentially linearly thereby imparting linear arrangement of the catheter throughout the pull-back. If the geometry of the organ footprint and or the medical device footprint does not have a "natural direction" because of central symmetry such as a circular footprint, then an additional GUI widget for an interactive marker d such as indicted on Fig 4 may be overlaid. Said maker allows defining the direction by mouse-click-and-drag or touch-and-swipe action (for touchscreen embodiments) or keyboard stroke. For example, in touch-and-swipe, the user's finger or a user operated stylus makes contact with screen M's surface at the screen area indicated by marker d (in other examples, a round-arrow or similar symbology suggestive of direction/orientation change is used). Upon finger-screen contact and whilst maintaining same, the user carries out a gesture for example tracing out an arc in the desired direction. Controller UIG operates to translate the touch events into a rotation angle. In this manner the interactive direction marker d may be rotated into the desired propagation direction and allows locking same (by double-touch finger "tap" or double mouse-click) for the current denervation procedure. The ablation point markers will then "pop up" on the screen one-by-one along the so specified direction. Whether or not the propagation direction can be determined automatically, the direction indictor d may be displayed at any rate to so leave to the user the option open to unlock and change the propagation direction.

In other words apparatus A acts to align the propagation direction of the markers in a clinically relevant manner that is based on organ and/or medical device position. In one embodiment, vessel RA segmentation is executed in the corresponding angiogram and the propagation d derived therefrom is the one used in the fluoro IM (assuming little motion at the targeted location, which is the case for renal denervation). As mentioned some embodiments rely on the wire in case of back-track ablation. In both cases a segmentation of the relevant vessel RA (or parts thereof or of device DC or of associated devices such as the catheter guidewire is executed.

According to one embodiment a virtual digital ruler or gauge DR is displayed alongside with the markers MP or MC. The graduations GR or scaling of the gauge is automatically adapted to the requirement of the current denervation procedure.

According to one embodiment and is shown in Figure 4 the gauge DR is represented by a double (or single) arrowed lead line with graduation GR that are represented by short line segments arranged vertical of said lead. Fig 4 shows a "ruler" embodiment of gauge DR.

The on-screen distances as demarked by any two consecutive ones of the markers or the DR graduations GR are so computed that moving the catheter so that footprint of catheter tip as displayed any of the fluoros advances from a current ablation point to the next displayed marker will result in the tip travelling in real the required ablation point clearance distance. To this end, the GUI controller uses current imager geometry, such as the SID and current screen resolution and or screen size to compute the correct scaling between the on-screen marker distances in respect of the real, required intra-ablation point distances. In another example the physical dimension of the ablation tip (which is extracted) can be used to determine with sufficient accuracy the mm/pixel relationship at the tip location. So, although the distances between the markers and between the graduations GR on the digital ruler DR can in some embodiments be shown at a scale 1:1, in other embodiments the on-screen distances will differ from the real distances travelled by the catheter DC. Graphical user interface controller GUIC as proposed herein will arrange the graduations and markers in such a manner that observing the on-screen distances/scalings will always result in the correct catheter tip T positioning throughout.

The scale used to place the graduations GR on gauge DR can be determined from the imager 100 system geometry (millimeter per pixel in the patient at the iso-center of the system: In a cone-beam geometry, there is a known homothetic relationship between pixel distances observed on the detector D and the corresponding real distances in the patient PAT. The homothetic factor is known at a given depth in the patient. In a C-arm system, the region of interest ROI is placed roughly at the iso-center of the imager system 100 so that, when the C-arm CA rotates, the ROI stays roughly centered in the image. The iso-center condition provides the depth information. In other embodiment the required scaling is determined from the image content, for instance, through the automatic measurement via segmentation of the diameter of the catheter guidewire or the dimension of the ablation-tip T which is known in advance and can be specified by the user at commencement of the intervention.

As mentioned earlier, GUI controller may operate in one embodiment in a "tracker" mode to further generate for display a marker for the current tip position T. This helps in positioning with high accuracy because operator can now focus on the task to move the catheter tip T so as to superimpose the current position marker with the marker for the next ablation point position MC. Said tracker marker may be rendered as a line orthogonal to the ruler and passing through the center of the catheter tip T.

The virtual gauge or ruler widget DR is positioned parallel to the renal artery. Since the denervation intervention is usually carried out under an Anterior-Posterior (AP) imager 100 angulation, the proximal part of the renal artery RV is usually viewable as a horizontal segment. In this embodiment, gauge DR is displayed horizontally across the image IM plane so it positioning is determined once gauge DR's location is determined. Using propagation direction indicator d may still be used to specify a tilted direction if thought appropriate. In any case, the location (and possible orientation) of the gauge DR can either be determined from a reference angiogram, or from the device DC's footprint in a fluoro IM. Since the ablation is performed from the distal to the proximal end of the targeted lesion during the pullback, the device DC is a reliable landmark at the start of the intervention. Usual segmentation techniques can be applied to segment those image objects.

Once the proximal part of the renal artery is identified (through direct vessel RA segmentation relying on the angiogram, or indirectly from segmentation in the relevant instance fluoro IM of the ablation device DC or its guidewire), the ruler DR is placed parallel to the vessel profile in a manner now described in more detail.

If the ruler DR is rectilinear it approximates, that is, it runs along the determined direction or orientation of vessel's RA or device's DC footprint in the respective image IM. This average direction is estimated as indicated earlier by a simple linear regression operation applied on the profile's direction curve (estimated by segmentation of vessel RA or the device DC), or from a pair of points such as i) the initial (at the start of the denervation procedure) ablation point and ii) an estimation of an anatomic landmark location such as the ostium OS location. In one embodiment, the later point is estimated relative to a related second medical device (for example the tip of the micro-catheter MC that is in place and is used to support the device DC's steering wire). In another embodiment, the second position to determine the direction d is to use the imager 100 geometry and assume a fixed direction, e.g. horizontal in case of the standard Antero-Posterior view for the renal artery. In case of a curvilinear embodiment of ruler DR (so as to represent and graphically mimic a "flexible" measuring tape), the ruler's profile runs or follows in parallel the vessel's profile (as obtainable from the angiogram) or the vessel's footprint, or from the device guidewire inside this vessel RA.

In both cases (rectilinear or curvilinear profiles), once the ruler DR's direction is estimated, there remains the task to determine ruler DR's position in the image IM plane. The position choice is at least driven partly by a two competing considerations: on the one hand the ruler should be close enough to the vessel or device so as to enable easy read off. On the other hand, it should not lie in the way of the intervention. In case of uncertain vessel RA profile, it should therefore be positioned with a pre-defined but user-configurable safety margin that allows for vessel RA's bending tilting, or other motions of same. When the vessel's profile is known with sufficient accuracy or confidence, and the motion is negligible, the ruler DR can be placed closer to the vessel so the user can instruct by user input the apparatus to apply a narrower safety margin. The decision on placing the ruler DR to the right or left hand side (relative to the direction d) of the vessel's footprint is application dependent and is again driven by ensuring that the most important anatomical details remain uncluttered and are not hidden by the overlay of the ruler DR graphics. In one embodiment, the ruler DR's position is adapted in response to table XB motion which is known by interrogating system 100 parameters and/or which can be estimated or derived from the image content by tracking bone landmarks for instance. Generally, if disturbing motion causes an overlap of the ruler DR overlay with the next ablation position, user interface generator UIG acts to apply a corresponding compensation so as to move the ruler away from the next intended (target) ablation point. Implementation of this "uncluttered view" feature can be achieved since the device DC is at least partly segmented (e.g., at least its ablation tip T) and the tip position can be closely monitored and based thereon the ruler DR's position can be changed accordingly. This operation also includes correcting the DR position for discrepancies between the angiogram and the fluoroscopy, in case where the vessel profile has been estimated by recourse to angiography support. User interaction is also envisaged in some embodiments so as to "drag-and-drop" the ruler DR widget in any suitable position or the user is to specify by touch or mouse click action a location anywhere on the image plane where the ruler DR should be placed.

In one embodiment, screen M is a touch screen and the markers can be shifted parallel to each other by finger touch-and-swiping action across screen M to allow further customizing, for example, by making the markers intersect (or by preventing intersection of) a desired on-screen feature. GUI controller ensures that the required intra-ablation point clearance is respected by restricting or constraining said shift to perpendicular directions relative to the propagation direction. So the shift can only occur "across" said predefined propagation direction. This embodiment is also envisaged for non-touch-screens, in which case the marker shift can be effected by a corresponding pointer tool event, for example, a click (on the marker in question)-and-drag action. In yet another embodiment it is the length of the markers that can be changed. For example, the marker can be made longer to have it extend to and intersect an image feature or to shorten said marker so as to prevent them to extend to the image feature for better visual inspection of said feature. Again, the maker length change may be effected by, as the case may be, a touchscreen touch-and-swipe action or by the mouse-click-and-drag action. Again, the direction of the length change is controlled by GUI controller UIG so as to maintain the correct intra-ablation point clearance. In other words, a user drag or swipe off the perpendicular direction will be automatically corrected by ignoring a swipe or drag component directed away from said perpendicular direction.

In another embodiment a length of the individual markers is automatically extended across the ruler and/or the catheter footprint.

According to one embodiment the GUI controller responds to a user request to fade-in for display all previous markers (with or without the latest next marker) together. This allows the user to check whether the applied ablation points are indeed "in-step" or "in sync" with required intra-ablation point distances, that is, the each of the markers passes through the respective one of the ablation point footprints as shown on the current/latest fluoro. By user "touch-and-swipe" action (in case screen M is a touchscreen) or by issuing a corresponding mouse click-and-drag event, the system of all previous markers can be shifted as a whole to fine-tune the fitting. A certain amount of patient movement can thereby be compensated for.

To sum up, apparatus A as proposed herein operates in some embodiments to automatically (that is, upon receipt of a ROI focused projection image IM) generate and effect display of a "virtual" digital gauge that will be automatically located at the right position sufficiently close and parallel to the renal artery RA footprint. In one embodiment, instead of, or in addition to the ruler, one or more markers are generated (crosshair lines or cursors) that represent the n-previous (n≥1) ablation point(s) (with or without an additional marker for the current position of the ablation tip) and/or the next target position. The one marker or more markers are positioned or are spaced apart from the immediately previous ablation point at the required ablation point clearance.

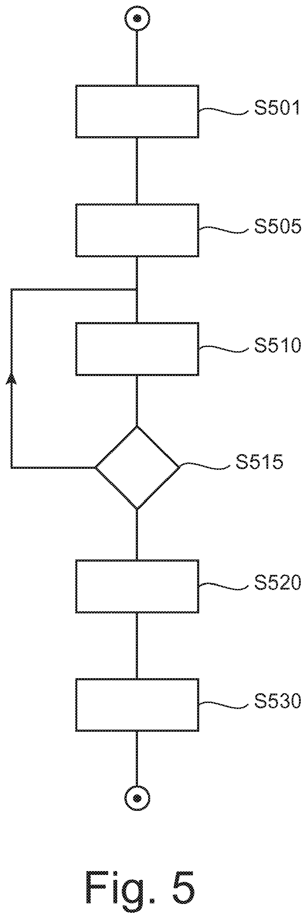

With reference to Figure 5 there is shown a flow chart for a method underlying in one embodiment operation of apparatus A.

At step S501 an X-ray projection image of an object or device in or around said object in the region of interest is received.

At step S505 a signal indicative of a current position and/or or point of action (for example ablation in a denervation) of a medical device DC (such as an energizable catheter's tip T) in or around said object (such as a renal artery) is received.

At step S510 a marker for display on a screen is generated and displayed overlaid on said image. The marker indicates the next point of action position for the device at a pre-determined distance from the detected current point of action of device DC.

At step S515 a second marker different from the first marker for a second point of action position at a second pre-determined distance from the previous next position is generated for display in response to receipt of a signal indicative of said second point of action position. In this manner in dynamic iterated fashion a new marker is generated and displayed indicating the relative next position as the device progresses through the object. The previous marker can be displayed alongside the respectively updated next marker position in one embodiment so that they are always at any time two markers displayed or only a single marker is displayed at any one time always indicating the respective next tip position.

At step S520 a direction for the arrangement of the markers on display is aligned with a detected orientation in the image of the medical device and/or the object in the image alignment updated changes with the change in object or device orientation.

At step S530 a virtual gauge is displayed overlaid on the image alongside with at least one of the markers to so enable the user to read off length information that relates to distances between the various device tip positions and/or the object such as the renal artery.

According to one embodiment the direction of the virtual gauge is automatically aligned with the device and/or object orientation, graduations on the gauge are automatically aligned the length of the object or device with a pre-defined of ablation point distances to a user provided or can be retrieved from a data base.

Apparatus A may be arranged as a dedicated FPGA or as hardwired standalone chip. However, this is an exemplary embodiment only.

In alternate embodiments, component A is resident on work station CON and runs thereon as one or more software routines. The components IN and UIG of apparatus A may be programmed in a suitable scientific computing platform such as Matlab® or Simulink® and then translated into C++ or C routines maintained in a library and linked when called on by work station CON.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An apparatus for supporting medical device positioning, comprising:
an input port (IN) for receiving
i) an X-ray projection image (IM) of an object (RA) and of a medical device (DC) and
ii) a signal indicative of a current application position of the medical device (DC) in or around said object (RA);
a user interface generator (UIG) configured to generate for display on a screen (M) a marker (MP) overlaid on said image, the marker (MP) indicating a next application position for the device (DC) at a first pre-determined distance from said current application position, the user interface generator (UIG) responsive to overlay a second marker (MC) for a second application position at a second pre-determined distance from the next application position upon receipt, at the input port (IN), of a signal indicative of said second application position.

2. Apparatus of claim 1, wherein the respective positions of the two markers (MP; MC) define a direction (d), the user interface generator (UIG) operative to automatically align said direction (d) with an orientation of the medical device (DC) and/or with an orientation of the object (RA), and operative to change or re-align said direction upon and in correspondence to a change in the object (RA) orientation or of the device (DC) orientation.

3. Apparatus of claim 1 or 2, wherein the X-ray projection image (IM) is acquired by an X-ray imager (100), the alignment and/or maker positioning operation of interface generator (UIG) based on a segmentation operation of an X-ray footprint (DCFP) of medical device (DC) in said image or of an X-ray footprint of the object (RA) in said image or in an object X-ray image.

4. Apparatus of any one of claims 1- 3, wherein the device issues said second signal whilst the device is or operates at the respective application position or wherein said second signal is issued upon the device reaching said next application position.

5. Apparatus of any one of claims 1- 4, wherein the at least one of the markers is a solid, dashed or dotted line or bar segment displayed so as to extend or run across said direction or wherein at least one of the two markers is displayed as any one or a combination of a cross-hair symbol, a chevron symbol, a circle, a dot.

6. Apparatus of any one of claims 1- 5, wherein the markers (MC, MP) are displayed alongside or wherein the second marker (MC) is displayed instead of the first marker (MP).

7. Apparatus of any one of claims 1- 6, wherein the user interface generator (UIG) is configured to generate for display on the screen a virtual gauge (DR) overlaid on the image alongside with at least one of the markers so as to enable a user to read off length information in relation to the object and/or the device and/or the at least one marker (MC), the virtual gauge (DR) including a lead line indicating said direction (d) on the screen.

8. Apparatus of claim 7, wherein the user interface generator (UIG) is configured to automatically align the virtual gauge (DR) with an orientation of the device (DR) or the object (RA) orientation and/or to automatically scale the length of and/or a graduation (GR) on the gauge (DR) in respective of a length of the object (RA) or of the device (DC).

9. Apparatus of any one of claims 1- 8, wherein the device (DC) is an energizable denervation catheter assembly (DS) suitable to denervate the object (RA) at one point at a time, each point corresponding to respective ones of the device application positions and the denervation catheter assembly issuing said signals upon energizing said denervation catheter (DC) assembly at the respective ones of the application positions.

10. Apparatus of any one of claims 1- 9, wherein the object (RA) is a renal artery of a human or animal patient (PAT).

11. A medical imaging system comprising:
an apparatus (A) of any one of previous claims 1-10;
an X-ray imager (100) supplying the image or images;
a screen (M) for displaying the image or images;
a denervation catheter assembly (DCA) or system for a denervation intervention supported by the image or images.

12. A computer-implemented method of supporting medical device positioning comprising the steps of:
receiving (S501, S505) i) an X-ray projection image of an object and ii) a signal indicative of a current application position of a medical device in or around said object;
generating (S510) for display on a screen a marker overlaid on said image, the marker indicating a next application position for the device at a first pre-determined distance from said current application position,
responsive to receiving a signal indicative of a second position, overlaying or displaying (S515) a second marker for the second application position at a second pre-determined distance from the next position.

13. Method of claim 12, wherein the respective positions of the two markers define a direction, the method further comprising:
automatically aligning (S520) said direction with an orientation of the medical device (DC) and/or with orientation of the object (RA), and operative to change the re-align said direction upon a change in the object or device direction.

14. A computer program element for controlling an apparatus according to any one of claims 1-11, which, when being executed by a processing unit is adapted to perform the method steps of claims 12-13.

15. A computer readable medium having stored thereon the program element of claim 14.

## Patentansprüche

1. Gerät zur Unterstützung der Positionierung einer medizinischen Vorrichtung, umfassend:
einen Eingangsanschluss (IN) zum Empfangen
i) eines Röntgenprojektionsbilds (IM) eines Objekts (RA) und einer medizinischen Vorrichtung (DC) und
ii) eines Signals, das eine aktuelle Anwendungsposition der medizinischen Vorrichtung (DC) in dem genannten Objekt (RA) oder um dieses herum angibt;
einen Benutzerschnittstellengenerator (UIG), der konfiguriert ist, um zur Anzeige auf einem Bildschirm (M) eine dem genannte Bild überlagerte Markierung (MP) zu generieren, wobei die Markierung (MP) eine nächste Anwendungsposition für die Vorrichtung (DC) in einem ersten vorgegebenen Abstand von der genannten aktuellen Anwendungsposition angibt, wobei der Benutzerschnittstellengenerator (UIG) bei Empfang, an dem Eingangsanschluss (IN), eines Signals, das eine zweite Anwendungsposition angibt, mit der Überlagerung einer zweiten Markierung (MC) für die genannte zweite Anwendungsposition in einem zweiten vorgegebenen Abstand von der nächsten Anwendungsposition reagiert.

2. Gerät nach Anspruch 1, wobei die jeweiligen Positionen der beiden Markierungen (MP; MC) eine Richtung (d) definieren, der Benutzerschnittstellengenerator (UIG) betriebsfähig ist, um die genannte Richtung (d) automatisch auf eine Orientierung der medizinischen Vorrichtung (DC) und/oder auf eine Orientierung des Objekts (RA) auszurichten, und betriebsfähig ist, um die genannte Richtung bei einer Änderung der Orientierung des Objekts (RA) oder der Orientierung der Vorrichtung (DC) und in Übereinstimmung hiermit neu auszurichten.

3. Gerät nach Anspruch 1 oder 2, wobei das Röntgenprojektionsbild (IM) durch einen Röntgenbildgeber (100) erfasst wird, wobei die Ausrichtungs- und/oder Markierungspositionierungsoperation des Schnittstellengenerators (UIG) basierend auf einer Segmentierungsoperation eines Röntgenfußabdrucks (DCFP) der medizinischen Vorrichtung (DC) in dem genannten Bild oder eines Röntgenfußabdrucks des Objekts (RA) in dem genannten Bild oder in einem Objektröntgenbild erfolgt.

4. Gerät nach einem der Ansprüche 1 bis 3, wobei die Vorrichtung das genannte zweite Signal ausgibt, während sich die Vorrichtung an der jeweiligen Anwendungsposition befindet oder dort betrieben wird, oder wobei das genannte zweite Signal ausgegeben wird, sobald die Vorrichtung die genannte nächste Anwendungsposition erreicht.

5. Gerät nach einem der Ansprüche 1 bis 4, wobei die mindestens eine der Markierungen eine durchgezogene, gestrichelte oder gepunktete Linie oder ein Balkensegment ist, die bzw. das so angezeigt wird, dass es sich in die genannte Richtung erstreckt oder quer hierzu verläuft, oder wobei mindestens eine der beiden Markierungen als ein Fadenkreuzsymbol, ein Winkelsymbol, ein Kreis, ein Punkt oder eine Kombination hiervon angezeigt wird.

6. Gerät nach einem der Ansprüche 1 bis 5, wobei die Markierungen (MC, MP) nebeneinander angezeigt werden oder wobei die zweite Markierung (MC) anstelle der ersten Markierung (MP) angezeigt wird.

7. Gerät nach einem der Ansprüche 1 bis 6, wobei der Benutzerschnittstellengenerator (UIG) konfiguriert ist, um zur Anzeige auf dem Bildschirm eine virtuelle Messlehre (DR) dem Bild überlagert zusammen mit mindestens einer der Markierungen zu generieren, um so einem Benutzer zu ermöglichen, Längeninformationen in Bezug auf das Objekt und/oder die Vorrichtung und/oder die mindestens eine Markierung (MC) abzulesen, wobei die virtuelle Messlehre (DR) eine Lotlinie einschließt, die die genannte Richtung (d) auf dem Bildschirm angibt.

8. Gerät nach Anspruch 7, wobei der Benutzerschnittstellengenerator (UIG) konfiguriert ist, um die virtuelle Messlehre (DR) automatisch auf eine Orientierung der Vorrichtung (DR) oder die Orientierung des Objekts (RA) auszurichten und/oder um die Länge der Messlehre (DR) und/oder ihre Gradeinteilung (GR) automatisch in Bezug auf eine Länge des Objekts (RA) oder der Vorrichtung (DC) zu skalieren.

9. Gerät nach einem der Ansprüche 1 bis 8, wobei die Vorrichtung (DC) eine energetisierbare Denervierungskatheterbaugruppe (DS) ist, die geeignet ist, um das Objekt (RA) zu einem Zeitpunkt zu denervieren, wobei jeder Punkt einer entsprechenden der Anwendungspositionen der Vorrichtung entspricht und die Denervierungskatheterbaugruppe die genannten Signale bei Energetisierung der genannten Denervierungskatheterbaugruppe (DC) an den betreffenden der Anwendungspositionen ausgibt.

10. Gerät nach einem der Ansprüche 1 bis 9, wobei das Objekt (RA) eine Nierenarterie eines menschlichen oder tierischen Patienten (PAT) ist.

11. Medizinisches Bildgebungssystem, umfassend:
ein Gerät (A) nach einem der vorhergehenden Ansprüche 1 bis 10;
einen Röntgenbildgeber (100), der das Bild oder die Bilder liefert;
einen Bildschirm (M) zum Anzeigen des Bilds oder der Bilder;
ein(e) Denervierungskatheterbaugruppe (DCA) oder -system für einen Denervierungseingriff unterstützt durch das Bild oder die Bilder.

12. Computerimplementiertes Verfahren zur Unterstützung der Positionierung einer medizinischen Vorrichtung, umfassend die folgenden Schritte:
Empfangen(S501, S505) i) eines Röntgenprojektionsbilds eines Objekts und ii) eines Signals, das eine aktuelle Anwendungsposition der medizinischen Vorrichtung in dem genannten Objekt oder um dieses herum angibt;
Generieren (S510), zur Anzeige auf einem Bildschirm, einer dem genannten Bild überlagerten Markierung, wobei die Markierung eine nächste Anwendungsposition für die Vorrichtung in einem ersten vorgegebenen Abstand von der genannten aktuellen Anwendungsposition angibt,
als Reaktion auf den Empfang eines Signals, das eine zweite Position angibt, Überlagern oder Anzeigen (S515) einer zweiten Markierung für die zweite Anwendungsposition in einem zweiten vorgegebenen Abstand von der nächsten Anwendungsposition.

13. Verfahren nach Anspruch 12, wobei die jeweiligen Positionen der beiden Markierungen eine Richtung definieren, wobei das Verfahren ferner umfasst:
automatisches Ausrichten (S520) der genannten Richtung auf eine Orientierung der medizinischen Vorrichtung (DC) und/oder auf eine Orientierung des Objekts (RA), und betriebsfähig, um die genannte Richtung bei einer Änderung in der Objekt- oder Vorrichtungsrichtung neu auszurichten.

14. Computerprogrammelement zum Steuern eines Geräts nach einem der Ansprüche 1 bis 11, das dafür ausgelegt ist, die Verfahrensschritte der Ansprüche 12 bis 13 durchzuführen, wenn es durch eine Verarbeitungseinheit ausgeführt wird.

15. Computerlesbares Medium, auf dem das Programmelement von Anspruch 14 gespeichert ist.

## Revendications

1. Appareil pour assister au positionnement d'un dispositif médical, comprenant :
un orifice d'entrée (IN) pour recevoir
i) une image de projection de rayons X (IM) d'un objet (RA) et un dispositif médical (DC)
et
ii) un signal indicatif d'une position d'application actuelle du dispositif médical (DC) dans ou autour dudit objet (RA) ;
un générateur d'interface utilisateur (UIG) configuré pour générer pour un affichage sur un écran (M) un marqueur (MP) superposé sur ladite image, le marqueur (MP) indiquant une position d'application suivante pour le dispositif (DC) à une première distance prédéterminée à partir de ladite position d'application actuelle, le générateur d'interface utilisateur (UIG) répondant à une superposition d'un second marqueur (MC) pour une seconde position d'application à une seconde distance prédéterminée de la position d'application suivante lors de la réception, au niveau de l'orifice d'entrée (IN), d'un signal indicatif de ladite seconde position d'application.

2. Appareil selon la revendication 1, dans lequel les positions respectives des deux marqueurs (MP ; MC) définissent une direction (d), le générateur d'interface utilisateur (UIG) fonctionnant de façon à aligner automatiquement ladite direction (d) avec une orientation du dispositif médical (DC) et/ou avec une orientation de l'objet (RA), et fonctionnant de façon à changer ou ré-aligner ladite direction par et en correspondance avec un changement d'orientation de l'objet (RA) ou de l'orientation du dispositif (DC).

3. Appareil selon la revendication 1 ou 2, dans lequel l'image de projection à rayons X (IM) est acquise par un dispositif d'imagerie à rayons X (100), l'alignement et/ou l'actionnement du positionnement du marqueur du générateur d'interface (UIG) sur la base d'une action de segmentation d'une empreinte de rayon X (DCFP) d'un dispositif médical (DC) dans ladite image ou d'une empreinte de rayon X de l'objet (RA) dans ladite image ou dans une image à rayon X de l'objet.

4. Appareil selon l'une quelconque des revendication 1 à 3, dans lequel le dispositif émet ledit second signal tandis que le dispositif est ou fonctionne à ladite position d'application respective ou dans lequel ledit second signal est émis lorsque le dispositif atteint ladite position d'application suivante.

5. Appareil selon l'une quelconque des revendication 1 à 4, dans lequel l'au moins un des marqueurs est une ligne continue, à tirets ou une ligne discontinue ou un segment de barre affiché de façon à s'étendre ou évoluer dans ladite direction ou dans lequel au moins l'un des deux marqueurs est affiché tel quel ou en combinaison avec un symbole en croix, un symbole en chevron, un cercle, un point.

6. Appareil selon l'une quelconque des revendication 1 à 5, dans lequel les marqueurs (MC, MP) sont affichés côte à côte ou dans lequel le second marqueur (MC) est affiché à la place du premier marqueur (MP).

7. Appareil selon l'une quelconque des revendication 1 à 6, dans lequel le générateur d'interface utilisateur (UIG) est configuré pour générer pour l'affichage sur l'écran, une mesure virtuelle (DR) superposée sur l'image côte à côte avec au moins l'un des marqueurs de façon à permettre à un utilisateur de lire des informations de longueur en relation avec l'objet et/ou le dispositif et/ou l'au moins un marqueur (MC), la mesure virtuelle (DR) incluant une ligne directrice indiquant ladite direction (d) sur l'écran.

8. Appareil selon la revendication 7, dans lequel le générateur d'interface utilisateur (UIG) est configuré pour aligner automatiquement la mesure virtuelle (DR) avec une orientation du dispositif (DR) ou une orientation de l'objet (RA) et/ou pour estimer automatiquement la longueur et/ou une graduation (GR) sur la mesure (DR) par rapport à une longueur de l'objet (RA) ou du dispositif (DC).

9. Appareil selon l'une quelconque des revendication 1 à 8, dans lequel le dispositif (DC) est un ensemble de cathéter de dénervation pouvant être excité (DS) approprié pour dénerver l'objet (RA) sur un point à la fois, chaque point correspondant à l'une respective des positions d'application du dispositif et l'ensemble de cathéter de dénervation émettant lesdits signaux lors d'une excitation dudit ensemble de cathéter de dénervation (DC) aux positions respectives des positions d'application.

10. Appareil selon l'une quelconque des revendication 1 à 9, dans lequel l'objet (RA) est une artère rénale d'un patient humain ou animal (PAT).

11. Système d'imagerie médicale comprenant :
un appareil (A) selon l'une quelconque des revendications 1 à 10 précédentes ;
un dispositif d'imagerie à rayons X (100) fournissant l'image ou les images ;
un écran (M) pour afficher l'image ou les images ;
un ensemble de cathéter de dénervation (DCA) ou un système d'intervention de dénervation assisté par l'image ou les images.

12. Procédé informatique d'assistance au positionnement d'un dispositif médical comprenant les étapes :
de réception (S501, S505) i) d'une image de projection à rayon X d'un objet et ii) d'un signal indicatif d'une position d'application actuelle d'un dispositif médical dans ou autour dudit objet ;
de génération (S510) pour un affichage sur un écran d'un marqueur superposé sur ladite image, le marqueur indiquant une position d'application suivante pour le dispositif à une première distance prédéterminée à partir de ladite position d'application actuelle,
répondant à la réception d'un signal indicatif d'une seconde position, de superposition ou d'affichage (S515) d'un second marqueur pour la seconde position d'application sur une seconde distance prédéterminée à partir de la position suivante.

13. Procédé selon la revendication 12, dans lequel les positions respectives des deux marqueurs définissent une direction, le procédé comprenant en outre :
un alignement automatique (S520) de ladite direction avec une orientation du dispositif médical (DC) et/ou avec une orientation de l'objet (RA), et fonctionnant de façon à changer ou ré-aligner ladite direction lors d'un changement de direction de l'objet ou du dispositif.

14. Elément de programme informatique pour commander un appareil selon l'une quelconque des revendications 1 à 11 qui, lorsqu'il est exécuté par une unité de traitement, est adapté pour réaliser les étapes du procédé des revendications 12 et 13.

15. Support pouvant être lu par un ordinateur ayant, stocké sur celui-ci, l'élément de programme selon la revendication 14.
